**Europäisches Patentamt**

(19) **European Patent Office**

**Office européen des brevets**

(11) Numéro de publication: **0 224 412 B1**

(12) **FASCICULE DE BREVET EUROPEEN**

(45) Date de publication du fascicule du brevet:
11.04.90

(51) Int. Cl.⁴: **C12N 9/52, C12P 21/02**

(21) Numéro de dépôt: 86402453.4

(22) Date de dépôt: 03.11.86

(54) Nouvel enzyme, son procédé d'obtention et son application à la préparation de N-(L-aspartyl-1)L-phénylalaninate de méthyle.

(30) Priorité: 05.11.85 FR 851365

(43) Date de publication de la demande:
03.06.87 Bulletin 87/23

(45) Mention de la délivrance du brevet:
11.04.90 Bulletin 90/15

(84) Etats contractants désignés:
DE GB NL

(56) Documents cités:
ANN. BIOL. ANIM. BIOCH. BIOPHYS.,
vol. 8, no. 4, 1968, pages 565-577; M.J. DESMAZEAUD et
al.: "Isolement, purification et propriétés d'une
protéase exocellulaire de Micrococcus caseolyticus"
EUR. J. BIOCHEM., vol. 19, 1971, pages 51-55; M.J.
DESMAZEAUD et al.: "Spécificité de la protéase neutre
de Micrococcus caseolyticus"

(73) Titulaire: SOCIETE FRANCAISE HOECHST Société
anonyme dite:, 3, avenue du Général de Gaulle,
F-92800 Puteaux(FR)

(72) Inventeur: Paul, François, 28 rue du Vivier, F-31650 Saint
Orens de Gameville(FR)
Inventeur: Duchiron, Francis, 58 chemin de Nichol,
F-31200 Toulouse(FR)
Inventeur: Monsan, Pierre, Renoufail Mondonville,
F-31700 Blagnac(FR)

(74) Mandataire: Rinuy, Santarelli, 14, avenue de la Grande
Armée, F-75017 Paris(FR)

## Description

La présente invention concerne un nouvel enzyme, son procédé d'obtention et son application à la préparation de N-(L-aspartyl-1) L-phénylalaninate de méthyle.

Actuellement, on utilise de plus en plus des procédés enzymatiques pour accéder à des substances présentant une activité physiologique. Ces procédés enzymatiques sont élégants et, très souvent, ils fournissent directement le produit désiré avec la configuration souhaitée sans qu'il soit nécessaire d'effectuer des séparations d'énantiomères ou de diastéréoisomères.

Le N-(L-aspartyl-1) L-phénylalaninate de méthyle, communément appelé aspartame, est un dipeptide très connu, doué de propriétés édulcorantes remarquables (R.H. Mazur et al., J. Amer. Chem. Soc., 1969, 91, 2684). Il peut être obtenu directement par des procédés enzymatiques à partir d'acide L-aspartique et de DL-phénylalaninate de méthyle (demandes de brevet européen No 0 0154 472, 0 124 313 et 0 074 095) mais la productivité de ces procédés reste faible. Aussi, prépare-t-on généralement ce composé soit par des procédés chimiques classiques, soit par coupure hydrogénante de son dérivé N-benzyloxycarbonyle issu de la condensation enzymatique du DL-phénylalaninate de méthyle avec l'acide L-N-benzyloxycarbonyl aspartique.

La Demanderesse a d'ailleurs mis au point un procédé de ce type, utilisant un système protéolytique, désigné SP, extrait d'un bouillon de culture de Micrococcus caseolyticus pour accéder à l'aspartame N-benzyloxycarbonyle à partir de l'acide L-N-benzyloxycarbonyl aspartique et de DL-phénylalaninate de méthyle.

Le Micrococcus caseolyticus, micro-organisme isolé à partir de lait de vache et dont une souche a été déposée dans la Collection Nationale de Micro-Organisme de l'Institut Pasteur à Paris sous le No 1194 le 28 avril 1982, fournit, en effet, sous certaines conditions de culture, un système protéolytique, SP, duquel on peut extraire une protéase unique, neutre, exocellulaire et protéolytique, de masse moléculaire comprise entre 35000 et 41000, désignée ci-après P, présentant la totalité de l'activité protéolytique du système protéolytique SP (M. Desmazeaud et al., Ann. Bio. Anim. Biochem. Biophys., 1968, 8, 419-429, 565-577 et European J. Biochem., 1971, 19, 51-55).

Or la Demanderesse a découvert dans ce système protéolytique SP un nouvel enzyme désigné ci-après E, démuni d'activité protéolytique sur la caséine et présentant, par ailleurs, la propriété de pouvoir condenser directement et sous certaines conditions l'acide L-aspartique avec le DL-phénylalaninate de méthyl pour fournir de l'aspartame.

La présente invention a donc pour objet ce nouvel enzyme E et un procédé permettant de l'obtenir.

L'enzyme E est une protéine présentant une masse moléculaire supérieure ou égale à 200 000 et, par électrophorèse en gradient de gel d'acrylamide dans des conditions dénaturantes, on constate que c'est un enzyme multimérique (une bande majeure vers 40 000 et deux bandes mineures vers 60 000). De plus, il n'hydrolyse pas la caséine.

On peut l'obtenir aisément par filtration sur gel (Séphacryl S 200 Superfine commercialisé par Pharmacia-France S.A., 78391 Bois d'Arcy, France) équilibré avec un tampon tris(hydroxy-éthyl)aminométhane (ci-après désigné TRIS)-chlorhydrate 20 mM pH 7,5 contenant du chlorure de calcium 1,5 mM, d'une solution du système protéolytique SP dans le même tampon avec élution au même tampon.

La filtration est suivie par mesure de la densité optique des éluats à 280 nm et les premières fractions correspondant à une masse moléculaire supérieure ou égale à 200 000 sont réunies puis lyophilisées. On isole ainsi l'enzyme E.

Le système protéolytique SP est connu et il peut être obtenu selon la technique décrite par M. Desmazeaud, en traitant par une solution aqueuse de sulfate d'ammonium un bouillon de culture à pH 7,0 de Micrococcus caseolyticus dont on a éliminé les bactéries par centrifugation puis en dissolvant le précipité obtenu dans une solution aqueuse de chlorure de sodium et enfin en lyophilisant cette solution préalablement soumise à une ultrafiltration. Ce système protéolytique est actuellement commercialisé par la société Roussel-Uclaf à Paris sous la dénomination "RULACTINE".

La présente invention a également pour objet une application de cet enzyme E à la préparation par voie enzymatique de l'aspartame.

Selon l'application de la présente invention, l'enzyme E, caractérisé par ses propriétés et par le procédé permettant de l'obtenir, offre la particularité étonnante de fournir économiquement et aisément de l'aspartame lorsqu'on le fait réagir sous les conditions habituelles aux procédés enzymatiques sur un mélange d'acide L-aspartique et de DL-phénylalaninate de méthyle.

Suivant cette application, on fait réagir à une température comprise entre 10 et 45°C et préférentiellement à 40°C, à un pH compris entre 4 et 8, avantageusement à un pH compris entre 5 et 7, en présence d'un mélange-tampon réglé à ce même pH, l'enzyme E sur une solution d'acide L-aspartique et de DL-phénylalaninate de méthyle, puis on isole l'aspartame formé par des moyens connus. Sous ces conditions réactionnelles, l'enzyme E réalise rapidement la condensation peptidique du carboxyle en position 1 de l'acide L-aspartique avec la fonction amine du L-phénylalaninate de méthyl pour fournir de l'aspartame. Selon des conditions avantageuses, on fait réagir une quantité d'enzyme E exprimée en protéines déterminées selon la méthode de Kalckar comprise entre 0,1 mg et 100 mg, préférentiellement entre 0,5 et 10 mg par millimole d'acide L-aspartique mis en oeuvre.

La formation de l'aspartame dans le milieu réactionnel peut être suivie en analysant périodiquement une prise d'essai par chromatographie liquide à haute pression (HPLC). Lorsque la concentration d'aspartame n'augmente plus de façon significative dans le milieu réactionnel, ce dernier est alors traité selon les méthodes habituelles pour isoler l'aspartame formé.

Selon une variante de l'application de la présente invention, on peut remplacer l'enzyme E par une quantité équivalente en enzyme E du système protéolytique SP d'où il est extrait. En effet, les autres enzymes présents dans ce système et notamment l'enzyme protéolytique P n'interfèrent pas avec l'enzyme E.

Les exemples suivants sont donnés à titre illustratif et nullement limitatif de la présente invention.

Exemple 1

1.1 Préparation du système protéolytique selon le mode opératoire tel que décrit dans le brevet francais N° 82,07883 publié sous le N° 2 525 865

Dans un fermenteur, on stérilise à 120°C une solution préparée à partir de 750 kg d'eau, 20 kg de corn steep liquide, 20 kg de peptone de caséine, 10 kg d'autolysat de levure et 906 g de chlorure de calcium dont le pH a été ajusté à 7 par addition de lessive de soude. On amène la température du milieu stérile à 30°C puis on introduit 15 litres d'une solution stérile de dextrose à 30 % et 10 litres de bouillon pied de cuve d'une culture de Micrococcus caseolyticus (souche N° 1194). On laisse fermenter 24 heures à 30°C sous agitation et air stérile en maintenant le pH constant pendant les dix premières heures. Après 24 heures, on recueille 1050 litres de bouillon brut de fermentation que l'on centrifuge pour éliminer les bactéries. On obtient ainsi 1000 litres de centrifugeat limpide. Dans ce centrifugeat, on ajoute 560 kg de sulfate d'ammonium, puis après 30 minutes d'agitation, 250 g d'Hyflosupercel. Après un repos de 24 heures, on élimine le milieu surnageant et on introduit la fraction insoluble obtenue dans 70 litres d'une solution de chlorure de calcium ; après 15 minutes d'agitation on filtre la solution obtenue, soit 82 litres, que l'on concentre par ultrafiltration. Après 12 heures, on obtient 10,5 litres d'une solution concentrée d'enzymes présentant une activité protéolytique de 50500 U par centimètre cube. Cette solution est ensuite lyophilisée. On isole ainsi 1 kg de système protéolytique SP présentant une activité protéolytique de 530 U par milligramme.

1.2. Dosage de l'activité protéolytique

L'activité protéolytique du système enzymatique est déterminée par mesure (variation de la densité optique à 280 nm) de la quantité de caséine hydrolysée par le système enzymatique (fraction non précipitable par l'acide trichloroacétique) dans des conditions standard. Pour ce faire, on détermine comparativement l'absorbance à 280 nm du filtrat de deux milieux réactionnels obtenus l'un en traitant sous agitation pendant 10 minutes à 30°C, 5 ml d'une solution de caséine à 1 % dans le tampon TRIS-HCl, 50 nM pH 7,5 contenant du chlorure de calcium 1,5 mM, par 50 microlitres d'une solution du système protéolytique SP à 2 g par litre dans le tampon TRIS 50 mM pH 7,5 contenant du chlorure de calcium 1,5 mM suivie de l'addition en fin de traitement de 5 ml d'une solution aqueuse d'acide trichloroacétique à 20 % et l'autre utilisé, en tant que témoin, obtenu en mélangeant au départ les mêmes réactifs y compris la solution aqueuse d'acide trichloroacétique. Une unité d'activité protéolytique, désignée U, correspond à une augmentation d'absorbance de 0,001 unité par minute.

1.3. Préparation de l'enzyme E

On dissout 1 g du système protéolytique SP contenant 270 mg de protéines totales (détermination par la formule de Kalckar) et présentant une activité protéolytique de 530 U/mg dans 0,1 litre de tampon TRIS-HCl 20 mM pH 7,5 contenant du chlorure de calcium 1,5 mM, puis cette solution est filtrée à la vitesse de 7,5 millilitres par heure et par centimètre carré de section de la colonne sur 3 litres de gel Sephacryl S 200 Superfine commercialisé par Pharmacia préparé au tampon TRIS-HCl 20 mM pH 7,5 contenant du chlorure de calcium 1,5 mM.

La filtration est suivie en mesurant l'absorbance des éluats à 280 nm et on recueille la première fraction correspondant à la protéine E de masse moléculaire supérieure ou égale à 200 000, puis en poursuivant l'élution, on recueille une deuxième fraction correspondant à la protéine neutre protéolytique exocellulaire, P, de masse moléculaire comprise entre 35 000 et 41 000, décrite par M. Desmazeaud et al. On détermine ensuite l'activité protéolytique sur la caséine des deux enzymes et l'on constate que l'enzyme E ne possède aucune activité protéolytique mais que celle-ci se retrouve en totalité dans l'enzyme P.

Après lyophilisation des éluats, on isole 53 mg de protéine E et 213 mg de protéine P (déterminations par la formule de Kalckar).

1.4. Préparation d'aspartame

On abandonne à 40°C pendant 2 heures une solution contenant :
- 20 mmoles d'acide L-aspartique,
- 20 mmoles de DL-phénylalaninate de méthyle,
- 26,5 mg de protéine E isolé précédemment, dans 100 ml de tampon MES-NaOH 100 mM pH 5,8, puis on introduit 5 ml d'acide chlorhydrique 2N pour arrêter la condensation et, par chromatographie liquide à haute pression sur une colonne micro Bondapack C 18 (commercialisée par Millipore Waters à Vélizy, France), détection UV à 254 nm et élution avec un mélange de tampon de phosphate de potassium 30 mM pH 6,8 - acétonitrile, 85/15 (V/V), on dose dans le milieu réactionnel 12 mg d'aspartame, soit 0,041 mmole. Note : MES désigne l'acide morpholino-2 éthanesulfonique.

Exemple 2

On reproduit l'exemple 1 en remplaçant l'enzyme E par une quantité équivalente du système protéolytique SP d'où cet enzyme E est extrait et l'on obtient comme précédemment de l'aspartame.

Exemple 3

On reproduit l'exemple 1 en remplaçant l'enzyme E par une quantité équivalente en protéine de l'enzyme P et l'on n'obtient pas d'aspartame.

Il va de soi que la présente invention n'a été décrite qu'à titre purement explicatif et nullement limitatif et que toute modification, notamment au niveau des équivalences, pourra y être apportée sans sortir de son cadre.

**Revendications**

1. Enzyme multimérique possédant une masse moléculaire supérieure ou égale à 200 000 et ne présentant aucune activité protéolytique sur la caséine, caractérisé en ce qu'il est issu d'une culture de Micrococcus caseolyticus.

2. Enzyme selon la revendication 1, caractérisé en ce qu'il est issu d'une culture de Micrococcus caseolyticus constitué par la souche N° 1194 déposée à l'Institut Pasteur.

3. Procédé d'obtention de l'enzyme selon la revendication 1 ou 2 par filtration sur gel équilibré par un tampon aqueux d'un système enzymatique préparé par lyophilisation d'un concentré aqueux issu de l'ultrafiltration de la solution obtenue par dissolution, dans une solution aqueuse de chlorure de calcium, d'un système enzymatique précipité d'un surnageant de bouillon de culture d'un micro-organisme par du sulfate d'ammonium, caractérisé en ce que l'enzyme n'est pas retenu sur un gel calibré pour des protéines de masse moléculaire comprise entre 5000 et 200000, équilibré avec un tampon TRIS-HCl 20 mM pH 7,5 contenant du chlorure de calcium 1,5 mM et que le système enzymatique est issu d'un bouillon de culture dudit Micrococcus caseolyticus.

4. Application de l'enzyme selon l'une quelconque des revendications 1 à 3 à l'obtention d'aspartame par condensation de l'acide L-aspartique avec le DL-phénylalaninate de méthyle.

**Claims**

1. A multimeric enzyme having a molecular weight higher than or equal to 200 000 and not exhibiting any proteolytic activity on casein, characterized in that the enzyme is derived from a culture of Micrococcus caseolyticus.

2. An enzyme according to claim 1, characterized in that the enzyme is derived from a culture of Micrococcus caseolyticus represented by strain No. 1194 deposited at the Institut Pasteur.

3. A method of producing the enzyme according to claims 1 or 2 by filtration on gel buffered with an aqueous buffer of an enzymatic system prepared by lyophilization of an aqueous concentrate formed by ultra-filtration of the solution obtained by dissolving, in an aqueous solution of calcium chloride, an enzymatic system precipitated from a supernatant of a microorganism culture broth by ammonium sulphate, characterized in that the enzyme is not retained on a gel calibrated for proteins with a molecular weight of between 5000 and 200 000, buffered with a 20 mM TRIS-GCl buffer, at pH 7.5, containing 1.5 mM calcium chloride, and the enzymatic system is derived from a culture broth of said Micrococcus caseolyticus.

4. Application of the enzyme according to any of claims 1 to 3 to the production of aspartame by condensation of L-aspartic acid with methyl DL-phenylalaninate.

**Patentansprüche**

1. Multimeres Enzym mit einer Molmasse größer oder gleich 200 000, das keinerlei proteolytische Aktivität gegenüber Kasein besitzt, dadurch gekennzeichnet, daß es aus einer Kultur von Micrococcus caseolyticus stammt.

2. Enzym nach Anspruch 1, dadurch gekennzeichnet, daß es aus einer durch den beim Institut Pasteur hinterlegten Stamm Nr. 1194 gebildeten Kultur von Mikrococcus caseolyticus stammt.

3. Verfahren zur Gewinnung des Enzyms nach Anspruch 1 oder 2 durch Filtration eines Enzymsystems über ein mit einer wäßrigen Pufferlösung äquilibriertes Gel, wobei das Enzymsystem durch Lyophilisation eines wäßrigen Konzentrates aus der Ultrafiltration der Lösung erhalten wurde, die durch Auflösung eines aus einem Überstand der Kulturbrühe eines Mikroorganismus mit Ammoniumsulfat ausgefällten Enzymsystems in einer wäßrigen Lösung von Calciumchlorid erhalten wurde, dadurch gekennzeichnet, daß das System nicht auf einem Gel zurückgehalten wird, das für Proteine mit einer Molmasse zwischen 5000 und 200 000 eingestellt und äquilibriert ist mit einer Pufferlösung TRIS-HCl 20 mM, pH 7,5, die 1,5 mM Calciumchlorid enthält und daß das Enzymsystem aus einer Kulturbrühe des Micrococcus caseolyticus stammt.

4. Verwendung des Enzyms nach einem der Ansprüche 1 bis 3 zur Herstellung von Aspartam durch Kondensation von L-Asparaginsäure mit D, L-Phenylalanin-Methylester.